# EUROPEAN PATENT APPLICATION

(11) **EP 4 491 831 A1**
(43) Date of publication of application: **15.01.2025**
(21) Application number: 23766823.1
(22) Date of filing: 07.03.2023
(51) Int. Cl.: E04G 21/02

(54) **METHOD FOR MANAGING CONCRETE CASTING AND WIRELESS COMMUNICATION DEVICE**

(30) Priority: 08.03.2022 JP 2022035337
(71) Applicant: Just.Will Co., Ltd., Fukuoka-Shi, Fukuoka 810-0022 (JP); The University of Tokyo, Bunkyo-ku, Tokyo 113-8654 (JP)
(72) Inventor: NOGUCHI, Takafumi, Tokyo 113-8654 (JP); NISHIJIMA, Shigeyuki, Fukuoka-shi, Fukuoka 810-0022 (JP); TSUKUI, Hiroshi, Fukuoka-shi, Fukuoka 810-0022 (JP); WANG, Cheng Hung, New TaipeiCity 24158 (TW); CHAN, Kin Yung, Taipei 10452 (TW)
(74) Representative: Winter, Brandl - Partnerschaft mbB
(86) International application number: PCT/JP2023/008487
(87) International publication number: WO 2023/171647

(57) **Abstract**

Provided is a wireless communication device can measure state quantity of concrete when a formwork is separated as long as strength of the concrete has reached to be predetermined strength, and faults of the concreted can be prevented even after the wireless communication device has been removed from the concrete. The wireless communication device includes: a temperature sensor measuring temperature of concrete within sheathing boards contacting therewith, and transmits signals of the temperature sensor by radio; a flange including a bottom surface flushing with a contact surface of an opening being formed in the sheathing boards; an insertion portion in a tapered conical shape protruding to an inside of the formwork from the flange, and holding the temperature sensor; a head portion with a diameter less than that of the flange exposing to an outside of the formwork from the flange, the wireless communication device is integrated with the concrete whereby: the head portion is fixed by means of a fixing tool until the formwork has not been separated; and the insertion portion is held by hardened and contracted concrete after the formwork has been separated.

## Description

### BACKGROUND OF THE INVENTION

### 1. Field of the Invention

The present invention relates to a wireless communication device for measuring temperature of concrete placed in a formwork in a construction site, and a method for managing concrete casting by means of the same.

### 2. Description of the Related Art

The applicants have already proposed and put into practical use an art wherein: temperature of a surface of concrete itself placed within a formwork is measured to able to perform separation of the formwork in accordance with objective raw data; and no foreign matter remains within the concrete after the formwork has been separated (See, for example, Reference 1: Japanese registered patent No. 5734785.).

The applicants also have proposed another art wherein a humidity term has been added in order to calculate the effective material age of concrete more precisely (See, Reference 2: Japanese patent application Laid-open No. 2022-11757).

In many cases, material age in 28 days after placing is often regarded as effective material age of concrete. In the system of the applicants, it is assumed that the sensor and the formwork are integrated and supported with each other. Therefore in the system of the applicants, there is a problem wherein statuses of the concrete cannot be measured after the formwork has been separated.

When using a method of mixing RFID chips each of which is provided with a sensor into the concrete, there is not such a limitation. Since a status wherein many RFID chips, which are foreign matters to the concrete and may cause faults (such as a crack) in the future, exist within the concrete should be avoided. In particular, it gets more serious when the RFID chips exist near the surface layer part of the concrete.

As shown in Reference 3 (Japanese Patent Application Laid-open No. 2001-13013), a case wherein a wired connection is assumed may be inconvenient, not realistic, and impractical. This is because a lot of formworks are used simultaneously on a construction site.

### [List of cited References]

Reference 1: Japanese registered patent No. 5734785
Reference 2: Japanese Patent Application Laid-open No. 2022-11757
Reference 3: Japanese Patent Application Laid-open No. 2001-13013

### OBJECTS AND SUMMARY OF THE INVENTION

In view of the above, an object of the present invention is to provide: a wireless communication device wherein state quantity of concrete can be measured in the same way not only when the formwork is assembled but also when the formwork has been separated until strength of the concrete has reached to be predetermined strength, and wherein faults of the concrete are prevented after separation of the formwork; and a method for managing concrete casting using the same wireless communication device.

A first aspect of the present invention provides a wireless communication device, comprising:
a temperature sensor for measuring temperature of concrete within a formwork contacting therewith, wherein the wireless communication device transmits signals of the temperature sensor by radio;
a flange including a bottom surface flushing with a contact surface of an opening being formed in sheathing boards constituting the formwork;
an insertion portion in a tapered conical shape protruding to an inside of the formwork from the flange, and holding the temperature sensor;
a head portion with a diameter less than that of the flange exposing to an outside of the formwork from the flange,
wherein:
   the wireless communication device is integrated with the concrete whereby the head portion is fixed by means of a fixing tool until the formwork has not been separated; and
   the wireless communication device is integrated with the concrete whereby hardened and contracted concrete holds the insertion portion after the formwork has been separated.

With this arrangement, first of all, an opening is formed to be prepared in at least one of a pair of sheathing boards constituting a formwork, and a flange of a wireless communication device is fit into the opening.

In this way, an insertion portion in a tapered conical shape is made to protrude from the flange of the wireless communication device to an inside of the formwork, and a head portion having a less diameter than a diameter of the flange is made to expose from the flange to an outside of the formwork.

When the above preparation has been made, concrete will now be placed within the formwork.

It is judged whether or not a formwork separation condition is fulfilled while measuring temperature of the concrete within the formwork by means of a temperature sensor stored within the insertion portion to maintain and keep the formwork assembled as long as the formwork separation condition is not fulfilled.

When the separation condition is fulfilled and the concrete within the formwork is hardened and contracted, the formwork is separated and removed. On the other hand, the insertion portion of the wireless communication device is kept to be held by the hardened and contracted concrete.

It is judged whether or not strength of the concrete has reached to be predetermined strength while measuring the temperature of the concrete by means of the temperature sensor, and the insertion portion is kept to be held by the hardened and contracted concrete as long as the strength has reached to be the predetermined strength.

After the strength of the concrete has reached to be the predetermined strength, the head portion of the wireless communication device is rotated and the insertion portion is separated from the concrete. In this way, the wireless communication device is removed from the concrete.

A second aspect of the present invention provides the wireless communication device, in addition to the first aspect, wherein the flange is in a disk-like shape.

With this arrangement, the flange can be made to fit into the opening of the formwork regardless of a rotating position of the head portion.

A third aspect of the present invention provides the wireless communication device, in addition to the first aspect, wherein a depth of the insertion portion is greater than a height of the head portion.

With this arrangement, the insertion portion can be reached to a deep layer part of the concrete to measure temperature of the same.

A fourth aspect of the present invention provides the wireless communication device, in addition to the first aspect, wherein a depth of the insertion portion is less than a height of the head portion.

With this arrangement, the insertion portion can be positioned in a surface layer of the concrete to measure temperature of the same.

In addition, it is preferable to fill and seal a filling material (such as a mortar) into a gap formed when the insertion portion has been removed after the wireless communication device has been removed from the concrete.

With this arrangement, the appearance of cast concrete can be improved.

In addition, it is preferable to the predetermined strength is design reference strength.

With this arrangement, operation in accordance with practical common sense can be made.

### Effect of Invention

As mentioned above according to the present invention, since the insertion portion of the wireless communication device is kept to be held by the hardened and contracted concrete not only when the formwork is assembled but also when the formwork is separated until the strength of the concrete has reached to be the predetermined strength, the state quantity of concrete can be continuously measured. In particular, even if a lot of formworks are used in parallel at the same site, measurement by radio can be made without hindrance.

Since a foreign matter does not remain within the concrete after having removed the wireless communication device from the concrete, faults (such as a crack) of the concrete can be prevented thereby.

### DETAILED DESCRIPTION OF THE PREFERRED EMBODIMENT S

Referring to the drawings, Embodiments according to the present invention will now be explained more concretely.

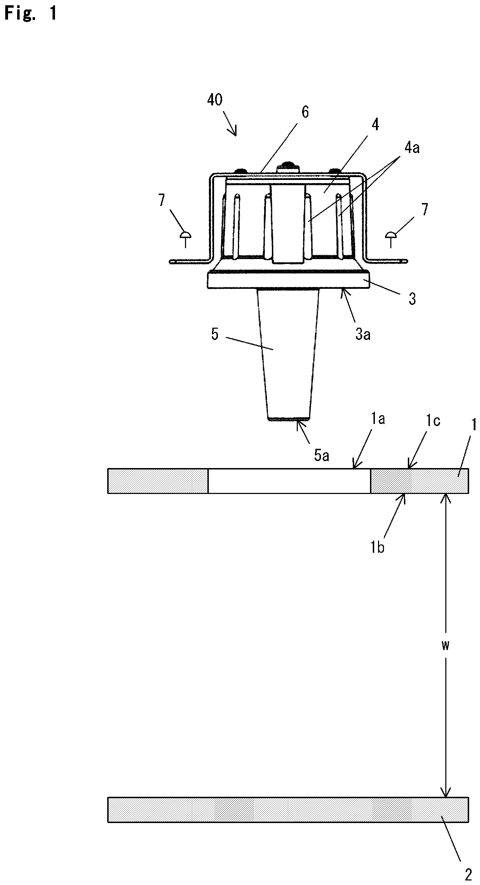

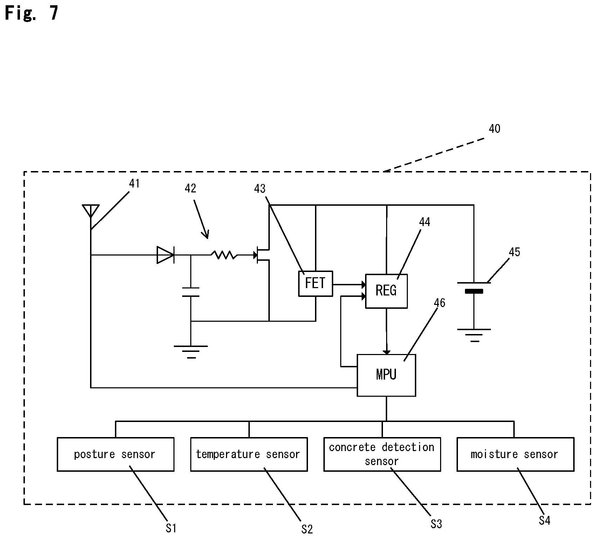

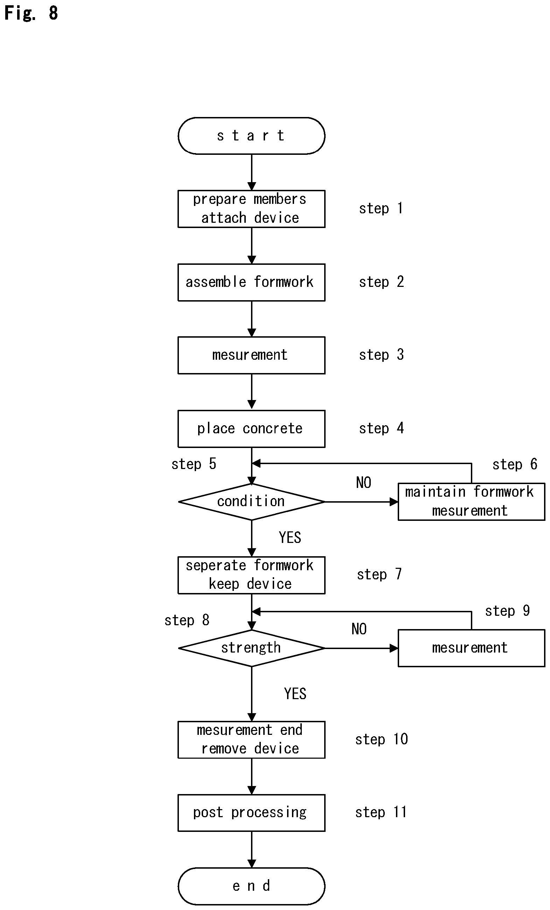

Fig. 1 through Fig. 6 are views showing a process of a method for managing concrete casting by means of a wireless communication device in Embodiment 1 according to the present invention. Fig. 7 is a block diagram of the wireless communication device in Embodiment 1 according to the present invention, and Fig. 8 is a flow chart showing the respective steps constituting the method for managing concrete casting by means of the wireless communication device in Embodiment 1 according to the present invention.

First of all referring to Fig. 7, an outline of the wireless communication device in this Embodiment will now be explained. In this Embodiment, a frequency of 13.56 [MHz] is used and RFID careers are transmitted according thereto. Of course, this does not mean that the frequency band is limited to the above-mentioned one, and another frequency band may be used instead thereof. Herein, the wireless communication device 40 includes the following elements.

The antenna 41 transmits and receives RFID careers, and the voltage application circuit 42 generates inductive voltage.

The FET (Field Effect Transistor) 43 is turned on when the voltage application circuit 42 generates the inductive voltage to make the regulator 44 operate.

When the regulator 44 begins to operate, it supplies electric power of the battery 45 to the micro processor 46.

In response to the supplied electric power, the micro processor 46 begins to control the respective elements of the wireless communication device 40. Herein, it is assumed in this Embodiment that the micro processor 46 includes a timer therein.

The following four sensors are provided in this Embodiment. The posture detection sensor S1 detects whether the posture of the formwork is horizontal or vertical. It is preferable to use an acceleration sensor as the posture detection sensor S1.

A mechanical type, an optical type, and a semiconductor type of acceleration sensor may be used. However, it is preferable to use the semiconductor type since this type is cheap and can be easily used.

It is preferable to use a three dimensional acceleration sensor. Alternatively, a one dimensional acceleration sensor and/or a two dimensional acceleration sensor may be used by devising installation positions and/or numbers thereof.

The temperature sensor S2 measures temperature of the placed concrete itself.

The concrete detection sensor S3 detects whether the placed concrete has reached to the position wherein the wireless communication device 40 is arranged.

The moisture sensor S4 detects state quantity with regard to moisture within the placed concrete.

Next referring to Fig. 8 and Fig. 1 through Fig. 6, processes of the method for managing concrete casting by means of the wireless communication device 40 of this Embodiment will now be explained.

First, as shown in Steps 1 and 2 of Fig. 8, members are prepared, the formwork is assembled, and the wireless communication device 40 is attached thereto. In addition, reinforcing bars are normally arranged within the formwork.

More concretely as shown in Fig. 1, the opening 1a, which penetrates in a thickness direction at least one (the first sheathing board 1) of a pair of the sheathing boards 1, 2 constituting the formwork, is formed. The interval of the pair of sheathing boards 1, 2 is set up considering the size W of the concrete structure to be build.

For convenience for showing the respective items in Figs., it will now be explained a case where the first sheathing board 1 is upper and the second sheathing board 2 is lower in order to construct a horizontal slab or the like.

However, the present invention can be also applied in the same manner to a case where the first sheathing board 1 is positioned in one of a left side and a right side and the second sheathing board 2 is positioned in the other of the left side and the right side one in order to construct a vertical column and/or a vertical wall. It should be clearly understood that the scope of the present invention involves such a case.

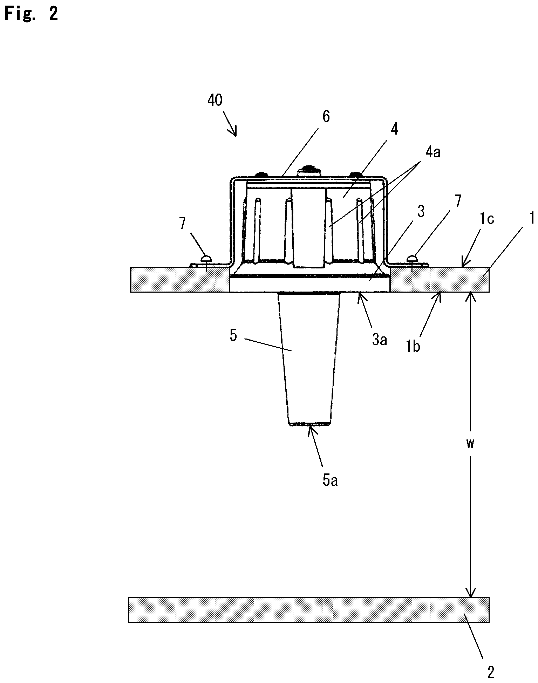

The wireless communication device 40 has an appearance as shown in Fig. 2, and includes the following elements.

It is preferable that the flange 3 is in a disk-like shape. The flange 3 includes a bottom surface flushing with of the bottom face 1b of the opening 1a formed in the first sheathing board 1. The flange may be formed in a shape of a rectangle, a polygon, or the like if there is no problem upon fitting it into the opening 1a of the first sheathing board 1 and/or removing it from the opening 1a of the first sheathing board 1.

The insertion portion 5 in a tapered conical shape protrudes from the flange 3 to an inside of the formwork.

The head portion 4 with a diameter less than that of the flange 3 exposes from the flange 3 to an outside of the formwork. Herein, the diameter of the head portion 4 is set up to be smaller than that of the flange 3. This is because such arrangement can make attaching the head portion 4 to the opening 1a of the first sheathing board 1 and detaching the same from the opening 1a of the first sheathing board 1 more easily. In order to prevent a hand of an operator from slipping during operation, it is preferable that the outer face of the head portion 4 is made to be a rough surface and/or protrusions 4a or the like are provided thereon as shown in Figs.

As shown in Fig. 1 to Fig. 2, after having fitted the flange 3 into the opening 1a, the wireless communication device 40 is fixed to the formwork by: attaching the screw 7 to the both ends of the fixing tool 6; and screwing the same into the first sheathing board 1.

This fixed state is continued until the formwork is separated, in other words, from when concrete is placed and soft until the concrete has been fully hardened and contracted. During this period, the wireless communication device 40 is integrated with the concrete whereby the head portion 4 is fixed to the first sheathing board 1 by means of the fixing tool 6.

In the situation of Fig. 2, measurement can be started as shown in Step 3 of Fig. 8.

More concretely, measured values by the respective sensors S1 to S4, or the like can be transmitted via the antenna 41 of Fig. 7 to external receivers (not shown in Figs., (e.g.) a smart phone, a personal computer, or the like.). Communications obvious to those skilled in the art can be performed. For example, the external receivers may store data thereon, and/or may transmit the data to a server at a predetermined site on the Internet, for example.

As shown in Step 4 of Fig. 8, placing concrete will now be started. Herein, Step 3 and Step 4 may be done simultaneously, or Step 4 may be done before Step 3. Vibration may be given to the concrete C by means of a vibrator. Otherwise, the vibration may not be given to the concrete C. Since there is no specific limitation with respect to a kind of concrete C, the kind of concrete C may be selected suitably.

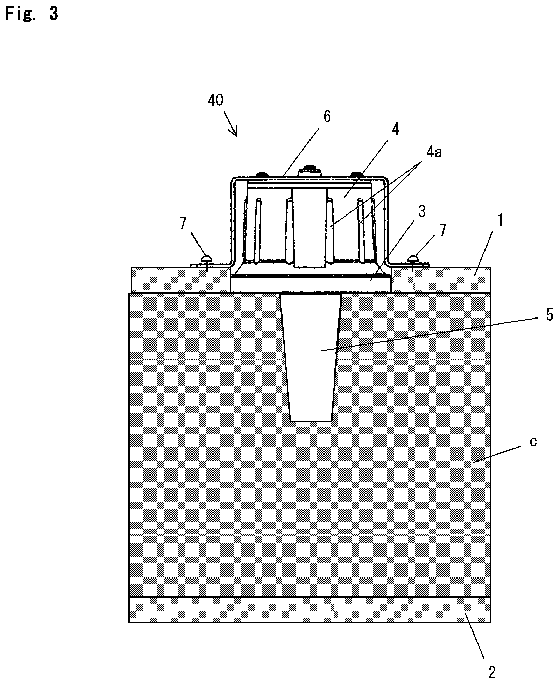

When the placed concrete has reached to the periphery of the wireless communication device 40, the situation is as shown in Fig. 3. Immediately after placing, the placed concrete is soft and in a gel-like state, after that the temperature of the placed concrete gradually increases as hydration reactions advance, and finally the placed concrete begins to be hardened and contracted.

At this stage as shown in Step 5 of Fig. 8, the MPU 46 keeps to judge whether or not a predetermined formwork separation condition is fulfilled, and the formwork is maintained to continue the measurement as long as the formwork separation condition is not fulfilled (Step 6).

When the formwork separation condition is fulfilled at Step 5, the screw 7 of the fixing tool 6 is loosened, and the fixing tool 6 is removed from the first sheathing board 1. Furthermore, the sheathing boards 1, 2 are also separated to disassemble the formwork. Alternatively, firstly the sheathing boards 1, 2 may be separated, and secondly the screw 7 of the fixing tool 6 may be loosened to remove the fixing tool 6 from the first sheathing board 1.

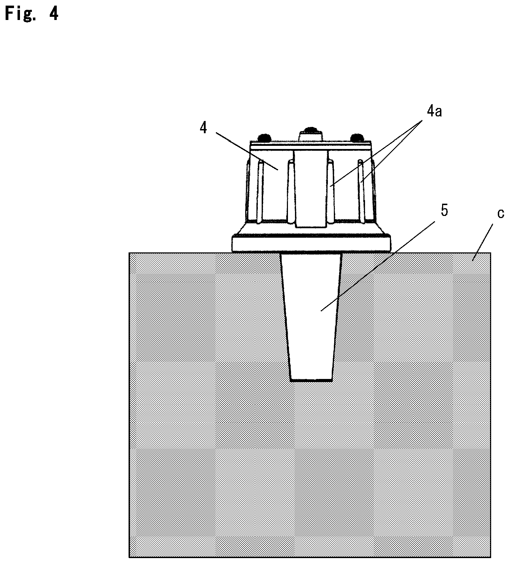

At this stage, the wireless communication device 40 is as shown in Fig. 4. The hardened and contracted concrete C itself strongly holds the insertion portion 5 of the wireless communication device 40. As a result, even after the sheathing boards 1, 2 have been separated and the formwork has been disassembled, the insertion portion 5 of the wireless communication device 40 remains to be coupled to the concrete C.

This relationship is in the same manner established in not only a first relation where the first sheathing board 1 is upper and the second sheathing board 2 is lower, that is to say the longitudinal direction of the insertion portion 5 is parallel to the direction of gravity, in but also a second relation where the first sheathing board 1 is in one of left and right directions and the second sheathing board 1 is in the other of the left and right directions, that is to say the longitudinal direction of the insertion portion 5 is perpendicular to or across the direction of gravity.

In fact, the experiments performed by the present inventors reveal that it is apparent that the insertion portion 5 of the wireless communication device 40 cannot be released easily from the hardened and contracted concrete C even in a case where the first sheathing board 1 is in the other of the left and right directions, since the insertion portion 5 of the wireless communication device 40 strongly connects to the hardened and contracted concrete C.

As shown in Steps 8 to 9 of Fig. 8, the measurement and communications performed by the wireless communication device 40 have been continued until the strength of the concrete C reaches to be design reference strength.

According to the present invention, the measurement and communications focusing on temperature history can be also performed even after the formwork is disassembled until the strength of the concrete C reaches to be the design reference strength.

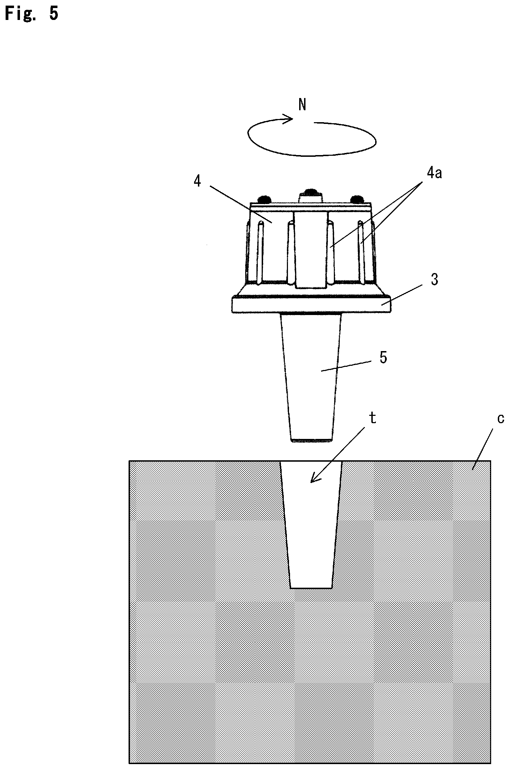

After having reached to be the predetermined strength as shown in Fig. 5, an operator grasps the head portion 4 by his/her hand to rotate the head portion 4 (for example, in the direction of arrow N), thereby removing the insertion portion 5 of the wireless communication device 40 from the concrete C. It depends upon the situation of the construction site when the strength of the concrete C reaches to be the predetermined strength. It may be 28 days after concrete placing, or may be less than/greater than 28 days. In order to make the separation easier, lubricant may be applied on an outer surface of the insertion portion 5 and/or a peeling layer or the like may be provided there-with.

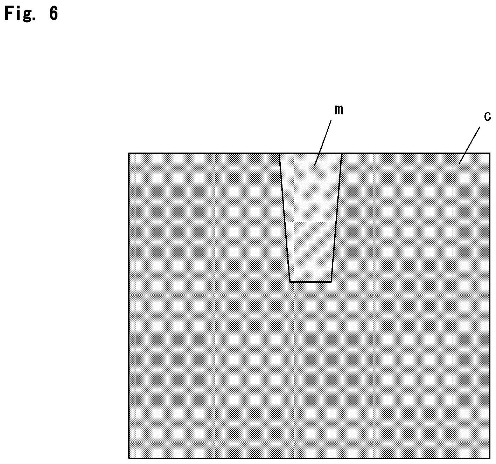

As a result of the separation, a portion where once the insertion portion 5 existed becomes to be a gap t. In order to improve an appearance of the placed concrete C as shown in Fig. 6, it is preferable to fill and seal a filling material (such as a mortar m) into the gap t.

According to the above explanation, it should be understood that a foreign matter does not remain within the concrete C after the wireless communication device 40 has been removed from the concrete C.

Hereinafter, two examples with respect to the length of the insertion portion 5 will now be explained.

### (Example 1)

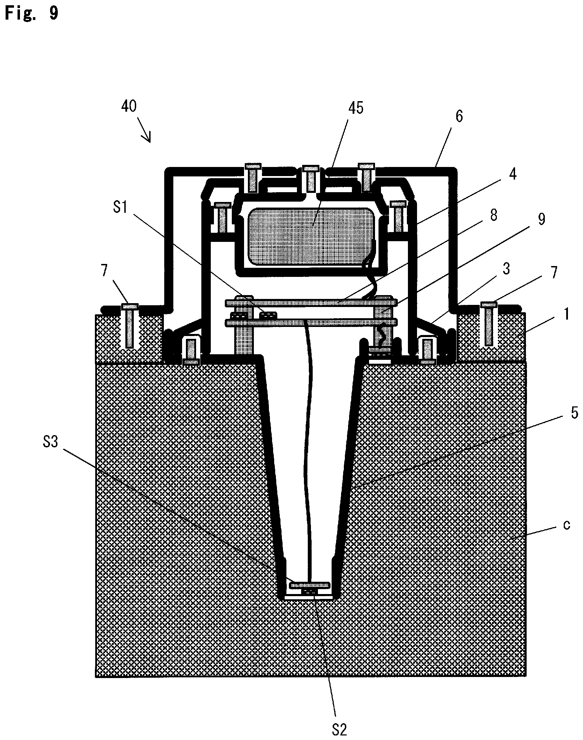

As shown in Fig. 9, in the wireless communication device 40 according to Example 1, the depth of the insertion portion 5 from the flange 3 is greater than the height of the head portion 4. Herein, the substrate 8, on which the circuit as shown in Fig. 7 is implemented, and the column 9 supporting the substrate 8 are provided within the head portion 4.

The relationship in Example 1 is suitable for measuring the deep layer part of the concrete C. The moisture sensor S4 may be omitted in the deep layer.

### (Example 2)

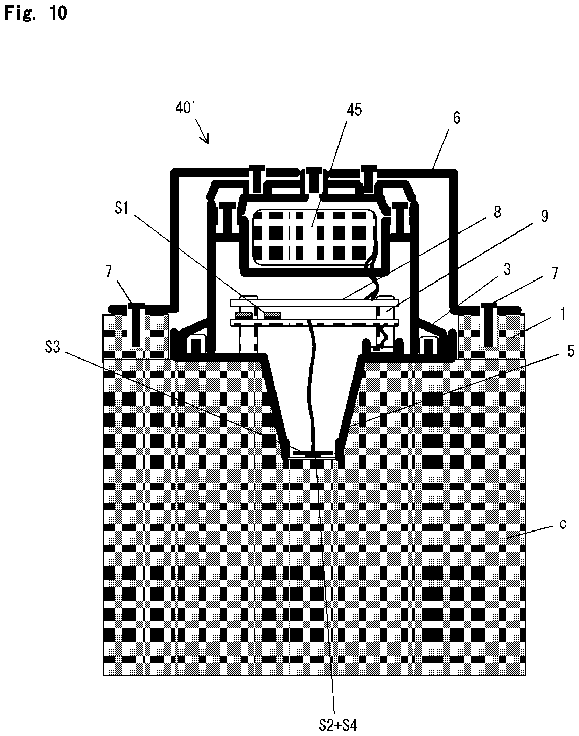

As shown in Fig. 10, in the wireless communication device 40' according to Example 2, the depth of the insertion portion 5 from the flange 3 is less than the height of the head portion 4. In this Example, it is preferable to provide the moisture sensor S4 in the almost same position as that of the temperature sensor S2. This is because drying tends to progress easily on the surface layer. After having removed the formwork, it is preferable to perform measurement by means of the moisture sensor S4.

Differing from Example 1, the relationship in Example 2 is suitable for measuring the surface layer of the concrete C.

### BRIEF DESCRIPTION OF THE DRAWINGS

Fig. 1 is a first view showing a process of a method for managing concrete casting by means of a wireless communication device in Embodiment 1 according to the present invention;
Fig. 2 is a second view showing a process of the method for managing concrete casting by means of the wireless communication device in Embodiment 1 according to the present invention;
Fig. 3 is a third view showing a process of the method for managing concrete casting by means of the wireless communication device in Embodiment 1 according to the present invention;
Fig. 4 is a fourth view showing a process of the method for managing concrete casting by means of the wireless communication device in Embodiment 1 according to the present invention;
Fig. 5 is a fifth view showing a process of the method for managing concrete casting by means of the wireless communication device in Embodiment 1 according to the present invention;
Fig. 6 is a sixth view showing a process of the method for managing concrete casting by means of the wireless communication device in Embodiment 1 according to the present invention;
Fig. 7 is a block diagram of the wireless communication device in Embodiment 1 according to the present invention;
Fig. 8 is a flow chart showing the method for managing concrete casting by means of the wireless communication device in Embodiment 1 according to the present invention;
Fig. 9 is a first sectional view showing a first insertion portion in Example 1 according to the present invention; and
Fig. 10 is a second sectional view showing a second insertion portion in Example 2 according to the present invention.

### BRIEF DESCRIPTION OF SYMBOLS

1, 2: Sheathing board
1a: Opening
1b: Contact surface
1c: Non-contact surface
3: Flange
3a: Bottom surface
4: Head Portion
4a: Protrusion
5: Insertion Portion
5a: Distal end portion
6: Fixing Tool
7: Screw
8: Substrate
9: Column
40, 40': wireless communication device
41: Antenna
42: Voltage application circuit
43: FET
44: Regulator
45: Battery
46: Micro processor
m: Mortar
t: Gap
S1: Posture detection sensor
S2: Temperature sensor
S3: Concrete detection sensor
S4: Moisture sensor

## Claims

1. A method for managing concrete casting, comprising:
a first step of forming an opening in at least one of a pair of sheathing boards constituting a formwork, and fitting a flange of a wireless communication device into the opening to make an insertion portion in a tapered conical shape protrude from the flange of the wireless communication device to an inside of the formwork, and to make a head portion having a less diameter than a diameter of the flange expose from the flange of the wireless communication device to an outside of the formwork;
a second step of placing concrete within the formwork;
a third step of judging whether or not a formwork separation condition is fulfilled while measuring temperature of the concrete within the formwork by means of a temperature sensor stored within the insertion portion to maintain the formwork as long as the formwork separation condition is not fulfilled;
a fourth step of removing the formwork in a state where the formwork separation condition is fulfilled and the concrete within the formwork has been hardened and contracted, and keeping the insertion portion held by the hardened and contracted concrete;
a fifth step of judging whether or not strength of the concrete has reached to be predetermined strength while measuring the temperature of the concrete by means of the temperature sensor, and keeping the insertion portion held by the hardened and contracted concrete as long as the strength has reached to be the predetermined strength; and
a sixth step of removing the wireless communication device from the hardened and contracted concrete whereby the head portion is rotated and the insertion portion is separated from the hardened and contracted concrete after the strength of the concrete has reached to be the predetermined strength.

2. The method for managing concrete casting as defined in claim 1, further comprising a seventh step of filling, after the sixth step, a filling material into a gap formed when the insertion portion of the wireless communication device has been separated.

3. The method for managing concrete casting as defined in claim 1 or 2, wherein the predetermined strength is design reference strength.

4. A wireless communication device, comprising:
a temperature sensor for measuring temperature of concrete within a formwork contacting therewith, wherein the wireless communication device transmits signals of the temperature sensor by radio;
a flange including a bottom surface flushing with a contact surface of an opening being formed in sheathing boards constituting the formwork;
an insertion portion in a tapered conical shape protruding to an inside of the formwork from the flange, and holding the temperature sensor;
a head portion with a diameter less than that of the flange exposing to an outside of the formwork from the flange,
wherein:
the wireless communication device is integrated with the concrete whereby the head portion is fixed by means of a fixing tool until the formwork has not been separated; and
the wireless communication device is integrated with the concrete whereby hardened and contracted concrete holds the insertion portion after the formwork has been separated.

5. The wireless communication device as defined in claim 4, wherein the wireless communication device is removed from the concrete whereby the head portion of the wireless communication device is rotated and the insertion portion is separated from the concrete after the concrete has reached to be predetermined strength.

6. The wireless communication device as defined in claim 4, wherein the flange is in a disk-like shape.

7. The wireless communication device as defined in claim 4, wherein a depth of the insertion portion is greater than a height of the head portion.

8. The wireless communication device as defined in claim 4, wherein a depth of the insertion portion is less than a height of the head portion.
